# EUROPEAN PATENT APPLICATION

(11) **EP 1 905 484 A1**
(43) Date of publication of application: **02.04.2008**
(21) Application number: 07123469.4
(22) Date of filing: 06.11.2002
(51) Int. Cl.: A61Q 19/00, A61K 8/39, A61K 8/36

(54) **Cosmetic composition and preparation method therefor**

(30) Priority: 30.11.2001 EP 01310049
(62) Divisional of application: 02777345.6
(71) Applicant: UNILEVER PLC, London, Greater London EC4 4BQ (GB); UNILEVER N.V., 3013 AL Rotterdam (NL)
(72) Inventor: Belmar, Maria, Teresa, Bedford, Bedfordshire MK44 1LQ (GB); Patrick, Alan, James, Bedford, Bedfordshire MK44 1LQ (GB); Zhu, Shiping, Bedford, Bedfordshire MK44 1LQ (GB)
(74) Representative: Acham, Nicholas Clive

(57) **Abstract**

A method of providing a topical base composition for use in the preparation of a topical skin care composition, comprising:
(a) providing a cosmetic container;
(b) providing in the container an effective amount of a fatty acid material having a melting point in the range of 40°C to 80°C; and a nonionic surfactant; this mixture being essentially anhydrous;
(c) providing sufficient heated water to the container such that substantially all of the fatty acid material is solubilised to provide a fatty acid/nonionic surfactant base mixture;
(d) agitating the contents of the container;
whereby a cream or lotion base is formed.

## Description

This invention relates to cosmetic compositions, and to preparation methods therefor. In particular, it relates to base compositions which may be used in cosmetic compositions such as skin and vanishing creams which may be rapidly prepared, for example, directly by a user.

Cosmetic compositions such as skin creams or lotions, and vanishing creams are well known. Such compositions are typically prepared on an industrial scale in a centralised manner, using large-scale equipment. In such a typical production process, for the production of products which are typically emulsions, the highest melting point ingredients are typically heated in a large container until they are molten (typically at around 80°C), stirred, and further ingredients are added to the composition as it is continually stirred and cooled. The last of these ingredients is often perfume, which may be added when the composition is typically at around 40°C. Thereafter, the composition is cooled and dosed into retail containers. The time and speed of shearing used in the process typically effects the droplet size of the resulting emulsion.

A number of problems may be associated with such large scale methods of manufacture. Foremost of these is the difficulty of individualisation or customisation of products, with the result that typically only relatively few products may be provided to the consumer. An example of this is the time and cost associated with changing products on a production line; because of the need to thoroughly clean the production apparatus between production runs of different products, especially with large scale manufacturing equipment, this contributes significantly to the difficulties of producing many different products on such a line within a reasonable time scale.

As a consequence of this, where a product is a customised or individualised product, and the batch of that particular product is relatively small, the cost of the product is relatively high.

In addition, a typical skin cream composition may easily comprise 60-90% water. As such, water makes up a large proportion of the weight of a topical product. It therefore represents a large proportion of the cost of transporting such a commercial product between manufacture and retailing.

Also, in many countries where incomes are relatively low, a consumer often cannot afford to purchase a large container of a cosmetic product. As such, there is high demand for cosmetic compositions which are provided in relatively small dosages, provided these can be made available at relatively low cost.

Yet a further problem associated with topical products is the instability of some of the more desirable ingredients of such products. An example of such an ingredient is retinol. Because retinol is relatively unstable, this leads to the situation where products containing retinol may have a relatively short shelf life, or alternatively a reduction in the functionality of retinol is experienced and accepted during the lifetime of the product. Where topical compositions do contain unstable components, it would be an advantage to be able to prepare them freshly.

FR-A-2649318 discloses the use of a pre-emulsion to overcome instability problems with certain ingredients and physical instability of the emulsion. The pre-emulsion is prepared from a final oil in water emulsion which is dehydrated. The resulting stock called pre-emulsion is reconstituted with water to form the final skin care composition. A disadvantage of this process is the high cost associated with forming an emulsion and then de-hydrating it.

Related to this, various desirable additives for skin care compositions are more stable and show optimum effect at lower pH, such as for example fatty acids, and in particular alpha hydroxy acids such as lactic acid and glycolic acid. Hence it is desirable for topical compositions containing such actives to have a relatively low pH (eg. 3.5-6 more preferably pH 3.5-4.5), in order to better stabilize such actives. Such pH is also relatively close to that of human skin. However, it can be difficult at times to provide a suitable base for a skin care composition having such a pH range.

We have found a method of manufacturing care compositions for topical application to the skin (such as, for example, skin creams, lotions, vanishing creams, sun tan lotions, moisturisers, and so on) which particularly lends itself not only to production of relatively small amounts of such compositions, but also compositions containing acidic (eg. alpha hydroxy acid) skin care actives, and in a relatively short time (e.g. a few minutes).

In its broadest aspect, it facilitates the manufacture of a base composition, especially in small amounts, which can be readily used to provide all manner of topical skin care compositions. Such a base composition can readily be customised by the addition of suitable active and emotive ingredients, to provide topical skin care compositions as required by the consumer. Such "small scale" production can encompass small industrial scale manufacture by a producer, or re-constitution by a consumer of a small amount of topical product from a concentrate.

Although the use of the base composition at small scale is highly preferred, it may also readily be used in an efficient large scale process such as at industrial scale.

Thus, according to a first aspect, there is provided a method of providing a base composition for use in the preparation of a cosmetic composition, especially a topical skin care composition, comprising:
(a) providing a cosmetic container;
(b) providing in the container an effective amount of fatty acid material having a melting point in the range 40-80°C and a nonionic surfactant; wherein the mixture of fatty acid material and nonionic surfactant is substantially anhydrous;
(c) providing sufficient heated water to the container such that substantially all of the fatty acid material is solubilised to provide a fatty acid/nonionic surfactant base mixture; and
(d) agitating the contents of the container;
whereby a cream or lotion base is formed.

The provision of an effective amount of fatty acid/nonionic surfactant base mixture most typically takes the form of the predosing in a container of a simple mixture of fatty acid material and nonionic surfactant. On the provision of heated water, the fatty acid/nonionic surfactant base mixture is formed.

We have found that merely mixing of fatty acid material and nonionic surfactant is required to form a suitable mixture in step (b). Preferably this mixing is at a temperature from 5 to 40 °C, even more preferred around room temperature without the use of any heating means. Room temperature is typically between 20 and 35 °C.

The provision of "heated water" in the container may also take several forms. In its simplest aspect, the heated water may be provided by simple dosing into the container of hot (eg. boiled) water at atmospheric pressure.
However other methods are envisaged; specifically envisaged is the dosing into the container of cold water, which is subsequently heated either by the application of microwave energy (from e.g. a domestic microwave oven), or the application of ultrasound.

One of the advantages of the current method is that there is no need to heat the mixture of fatty acid material and nonionic surfactant. Only the added water phase is heated and then mixed with the composition of step (b) which is preferably at room temperature (20 to 35 °) or lower. This already leads to the formation of a suitable cream or lotion base.

Therefore in a preferred embodiment, the mixing in step (b) is by mechanical mixing at a temperature from 5 to 40 °C. Even more preferred the heated water is added to the mixture of step (b) while the mixture of step (b) is at a temperature from 5 to 40 °C, preferably from 20 to 35 °C, more preferred from 20 to 30°C.

The cream or lotion base formed is preferably a stable product as such and may be applied to the skin. This product differs from cloudiness agents such as those disclosed in JP-A-04297412.

By "solubilised" in this context is meant a possible combination of dissolving and/or melting the fatty acid.

Conveniently, the anhydrous composition in the container is a simple mechanical mixture, and may conveniently be in the form of a tablet, powder or amorphous mass. Hence in another form of the invention, there is provided an anhydrous composition in the form of a tablet, powder or amorphous mass for dosing into a container, for use according to the invention. Such an anhydrous composition may be provided separately to the container in which the cosmetic composition may be prepared, and may be individually wrapped.

Where the container is a cosmetic container and the preparation is being carried out for the preparation of an individual portion of the skin care composition by a consumer, it will be necessary to seal the cosmetic container prior to agitation. Such cosmetic containers may preferably have a volume of 20-250ml, more preferably 25-100ml, even more preferably 25-50ml.
Even then, it is preferred that in such containers the volume of contents (eg. water, base materials, and other components necessary to make the topical composition) do not make up more than 50% of the volume of the container, so as to allow sufficient room for thorough mixing of the contents during agitation.

The optimum size and shape for the container can easily be determined by routine simple experimentation. However, the optimum container will provide for both rapid and thorough solubilisation by the heated water of the fatty acid material, but also rapid heat loss. This second feature is significant, since ideally agitation of the container is continued until the contents of the container are "set", e.g. they do not readily splash around the container during shaking.

The dimensions of the "container" are significant in ensuring that thorough solubilising of the fatty acid material occurs. When the embodiment of the invention lies in the provision of a container to the consumer which contains the anhydrous base ingredients and other components necessary for a topical skin composition, this will have consequences for the dimensions and size of the container. However, given that the invention is also suitable for small scale industrial production, the "container" and its dimensions may relate to the appropriate mixing chamber in a batchwise or continuous mixing apparatus. In particular, as well as the making up of individual compositions according to the invention by the user at home, the invention also lends itself to the small scale manufacture of compositions, for example at the point of sale in shops and beauty parlours, using pilot plant machinery, and in mobile vending facilities which are common in various parts of the world.

It is preferred that the base composition resulting in step (b) is taken to a remote location before the addition of water to form the lotion or cream base. In case the composition of step (b) is applied in a (small scale) industrial process the composition may be stored as such and used in a further process and transported to a separate vessel for continuation with step (c).

According to a further aspect of the invention, there is provided a re-sealable cosmetic container containing an anhydrous composition comprising a fatty acid material having a melting point in the range 40°C to 80°C, and a nonionic surfactant.

By "anhydrous" in this context is meant containing less than 5% by weight of water, preferably less than 2% water, even more preferably less than 1% water, and ideally less than 0.5% water.

The method and packaged composition of the invention provide a surprisingly effective method of manufacturing a cosmetic composition base for a topical skin application, which base material lends itself both to rapid small scale production, and also customisation with such further ingredients or additives as may be necessary to provide the desired effective topical composition. Such extra ingredients necessary for the formulation of the final topical composition may conveniently (but not essentially) be pre-dosed into the container along with the fatty acid material and the nonionic surfactant.

Although not wishing to be bound by theory, the essence of the invention is thought to lie in the provision of heated water to a mixture of nonionic surfactant and a fatty acid material to provide a fatty acid/nonionic surfactant base mixture, which mixture combined with the water provides a base for a cream or lotion which has desirable physical and rheological properties. In practising the method of the invention, the amount and temperature of the heated water added to the container should be sufficient to cause substantially all of the fatty acid material in the container to melt. This facilitates the formation of the appropriate crystal structure to provide the base with desirable rheological properties.

Compositions according to the invention have typically been found to have a slightly unusual net-type structure, with bridging links visible under a microscope between adjacent plates of material. The resulting compositions have also been found to have unusual sensory properties, having a relatively draggy, and occasionally slightly tacky feel. The compositions also tend to be characterised by fairly quick absorption to the skin, and low levels of residual whiteness.

The small scale nature of the invention is important and therefore preferred, since it is especially by the provision of a relatively small container that substantially all of the fatty acid material can be readily melted using only heated water, and secondly that the ingredients of the topical composition can be sheared by a regime (e.g. shaking) which does not require specialist shearing equipment.

Also, the cooling of the product is quick without the need for specialist equipment.

That said, in certain embodiments it may be possible and indeed desirable to use shearing equipment.

A further important aspect of the invention lies in that the relative amounts of ingredients of the fatty acid material, nonionic surfactant and water are determined and used to ensure the formation of the appropriate fatty acid/nonionic surfactant base mixture. The resulting fatty acid/nonionic surfactant base mixture provides desirable rheological properties for the resulting base product, which properties can be manipulated inter alia by varying the relative amounts of the starting ingredients, and hence the relative amounts of fatty acid material and nonionic surfactant in the base material.

There are other methods of manipulating the final properties of the compositions according to the present invention. For example, in an envisaged aspect, if the container is subjected to ultrasound as part of the agitation step, the resulting cosmetic product may be slightly harder than otherwise.

To ensure formation of the appropriate crystal structure and sufficient melting/dissolving of the fatty acid nonionic surfactant base mixture, and also avoid premature reaction, it is preferable that the ingredients of the fatty acid/nonionic surfactant base mixture be substantially anhydrous prior to the provision of heated water. Once the heated water has been provided to the container, agitation of the water and the ingredient materials is required for homogeneity. Where the container is a re-sealable (e.g. screw top) container in which a consumer is making an individual portion of skin composition, that agitation is conveniently provided by shaking the sealed container, which should provide sufficient agitation.

Conveniently, the container is agitated for a period of at least 10 seconds, more preferably at least 20 seconds, even more preferably at least 30 seconds. Conveniently agitation may last up to 5 minutes. Conveniently, agitation is continued until at least the time that the contents no longer splash around the container during agitation.

It is also important that the temperature and amount of the heated water used be sufficient to melt and/or dissolve substantially all of the fatty acid material in the container. The minimum temperature of the hot water required will depend, amongst other things, on the amount of water used, but the heated water will typically have a temperature of at least 80°C, preferably at least 85 °C, more preferably at least 90°C, even more preferably at least 95°C and may even be boiling.

An essential element of the invention is the fatty acid material. "Fatty acid material" may include blends of fatty acids, typically containing fatty acid moieties with chain lengths of from C₈ to C₂₂. The fatty acid material may also contain relatively pure amounts of one chain length fatty acid moiety. Suitable fatty acids from which fatty acid/nonionic surfactant base mixtures may be derived include pelargonic, lauric, myristic, palmitic, stearic, isostearic, oleic, linoleic, ricinoleic, arachidic, behenic and erucic acids. Although normally saturated, suitable fatty acid materials may contain unsaturated fatty acid moieties, and may contain fatty acid moieties having a degree of substitution, such as e.g. hydroxy fatty acids. Melting points of the individual fatty acids are also relatively unimportant; what is most important is the overall melting point of the fatty acid material, which should be in the range 40°C to 80°C In certain preferred embodiments, the fatty acid material will contain relatively high amounts (e.g. at least 50%, preferably at least 75%) of stearic and/or palmitic acid moieties. The chain length of the fatty acid material used according to the invention will influence the minimum temperature of the heated water needed to be used in the invention, since an important aspect of the invention is the fairly thorough melting of the fatty acid material on the provision of the heated water. However, the chain length of the components of the fatty acid material also determines the rheological properties of the resultant skin composition base. A fatty acid material mix containing relatively high proportions of stearic and palmitic acid moieties has been found to be particularly suitable for use for manufacturing skin creams and lotions which may be used in temperate to hot climates.

However, fatty acid material mixtures containing relatively high amounts of lower chain length fatty acid moieties (e.g. more than 50% of the fatty acid moiety having a chain length of C₈-C₁₄) may also be suitable for the preparation of skin compositions for use in relatively cold climates.

Topical cosmetic compositions according to the invention typically have a pH in the region 3.5 to 6.5, preferably 3.5 to 6, even more preferably 3.5-4.5.

Compositions according to the invention also include as a starting ingredient a nonionic surfactant. Most nonionic surfactants are thought to be suitable. However, particularly preferred nonionic surfactants are polyoxyethylene ester and ether surfactants, a specific example of which is Tween 60. Other suitable nonionic surfactants are described in McCutcheons "Detergents and emulsifiers", North American edition (1986), published by Allured Publishing Corporation, the contents of which are specifically incorporated herein by reference.

Little is known about the structure of the cosmetic base compositions formed according to the invention by mixing the fatty acid material and the nonionic surfactant. However the resultant base material formed after the provision of hot water often has a characteristic net type structure when viewed under a microscope, with thin bridging links visible between adjacent crystalline plates.

Base compositions formed according to preferred embodiments of the invention are made from a mixture of fatty acid material and nonionic surfactant, the ratio of the two being carefully controlled to optimise physical and sensory properties.

Typically, the ingredients of the fatty acid/nonionic surfactant base mixture are adjusted so as to provide a base mixture comprising a weight ratio of nonionic surfactant to fatty acid material of 1:1 to 1:20, more preferably 1:2 to 1:10, even more preferably 1:5 to 1:10. In certain embodiments, particularly suitable skin care composition bases have been achieved when the ratio of nonionic surfactant to fatty acid material is around 1:7.

In a preferred embodiment, the invention may comprise a re-sealable cosmetic container containing a "concentrate", which may be dry, and may conveniently be in the form of a tablet, dried powder or amorphous mass, which concentrate is for the provision of a cosmetic, especially skin care composition after reconstitution with hot water. The concentrate may contain the essential base materials, i.e. a fatty acid material and nonionic surfactant, and any other additives which are necessary or desirable to form the eventual cosmetic composition, especially the topical skin care cream or lotion.

Compositions according to the invention are particularly suited as base materials to provide skin care creams or lotions. In some circumstances, the resultant fatty acid/nonionic surfactant base mixture formed after reconstitution could provide a suitable topical skin care composition on its own. However, it is normally desirable to add further ingredients to the composition to provide the final skin care composition.

Resulting topical compositions according to the invention may typically contain 50% to 90% water, more preferably 70% to 85% water, and sufficient fatty acid material and nonionic surfactant to provide the desired structure. Suitable levels of fatty acid are generally in the range 5% to 25%, more preferably 10% to 20%, and suitable levels of nonionic surfactant are generally in the range 0.5% to 10%, more preferably 2% to 8%.

Examples of suitable additives for cosmetic compositions, especially skin care compositions are given below.

Besides water, relatively volatile solvents such as e.g. C₁₋₃ monohydric alcohols may be part of the cosmetic vehicle of composition, though they are usually present only at relatively low levels since they tend to evaporate when hot water is added to the base concentrate.

Topical cosmetic composition according to the invention typically comprise 60-99% of aqueous cosmetic base (eg. water, fatty acid and nonionic surfactant), the balance comprising other components (additives) necessary to provide the desired form of topical skin care composition.

Particularly preferred additives in topical compositions according to the invention are skin care actives which are acidic in aqueous solution, such as for example fatty acids, especially alpha hydroxy fatty acids; as well as peroxides such as hydrogen peroxide, vitamins such as vitamin B3, and poly saccharides such as chitosan; particularly preferred alpha hydroxy fatty acids are lactic acid and glycolic acid.

Emollient materials may also serve as cosmetically acceptable additives. These may be in the form of silicone oils and synthetic esters. Amounts of the emollients may range anywhere from 0.1 to 5%, preferably between 1 and 20% by weight of the final topical skin care composition.

The emollient material may be a silicone oil, an ester or a mixture of these.

Silicone oils may be divided into the volatile and non-volatile variety. The term "volatile" as used herein refers to those materials which have a measurable vapour pressure at ambient temperature. Volatile silicone oils are preferably chosen from cyclic or linear polydimethyl siloxanes containing from 3 to 9, preferably from 4 to 5 silicon atoms. Linear volatile silicone materials generally have viscosities less than about 5 centistokes at 25°C, whilst cyclic materials typically have viscosities of less than about 10 centistokes.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 25 million centistokes at 25°C. Among the preferred non-volatile silicone emollients useful in the present compositions are the polydimethyl siloxanes having viscosities from about 10 to about 400 centistokes at 25°C.

Among the ester emollients are:
(1) Alkenyl or alkyl esters of fatty acids having 10 to 20 carbon atoms. Examples thereof include isoarachidyl neopentanoate, isononyl isononanoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate and arachidyl behenate.
(5) Sterol esters of which cholesterol fatty acid esters are examples thereof.

Humectants of the polyhydric alcohol type may also be employed as part of the cosmetic vehicle in cosmetic compositions, especially skin care compositions of this invention. The humectant aids in increasing the effectiveness of the emollient, reduces scaling, stimulates removal of built-up scale and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. The amount of humectant may range anywhere from 0.5 to 30%, preferably between 1 and 15% by weight of the final skin care composition.

Compositions according to the invention may beneficially comprise little or no additional thickener. However, if desired, thickeners (in minor amounts) may also be utilised as part of the cosmetically acceptable vehicle of compositions according to the present invention. Suitable thickeners include cross-linked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0001 to 2%, usually from 0.001 to 1%, by weight of the composition, if at all.
An oil or oily material may be present, together with an emulsifier to provide typically oil-in-water emulsion, though this will depend largely on the average hydrophilic-lipophilic balance (HLB) of the emulsifier employed.

Various types of additional active ingredients may be present in cosmetic compositions of the present invention. Actives are defined as (skin) benefit agents other than emollients and other than ingredients that merely improve
the physical characteristics of the composition. Although not limited to this category, general examples include additional anti-sebum ingredients such as talcs and silicas, and sunscreens.

Sunscreens include those materials commonly employed to block ultraviolet light. Illustrative compounds are the derivatives of PAA, cinnamate and salicylate. For example, azobenzophenone (Parsol 1789®) octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks Parsol MCX and Benzophenone-3 respectively. The exact amount of sunscreen employed in the compositions can vary depending upon the degree of protection desired from the sun's UV radiation.

Compositions may also contain other surfactants.

Many cosmetic compositions, especially those containing water, must be protected against the growth of potentially harmful micro-organisms. Preservatives may therefore be necessary, though because of the essentially anhydrous nature of the concentrate, the need for preservatives may be reduced.

Suitable preservatives include alkyl esters of p-hydroxybenzoic acid, hydantoin derivatives, propionate salts, and a variety of quaternary ammonium compounds. Particularly preferred preservatives of this invention are methyl paraben, propyl paraben, phenoxyethanol and benzyl alcohol. Preservatives will usually be employed in amounts ranging from about 0.1% to 2% by weight of the final skin care composition.

Cosmetic compositions, especially skin care compositions according to the invention may also include a retinoid. Retinoids increase collagen synthesis by dermal fibroblasts. This results in protection from sun damage and smoothening of wrinkled skin. The term "retinoids" as used herein, includes retinoic acid, retinol, retinal, and retinyl esters. Included in the term "retinoic acid" are 13-cis retinoic acid and all-trans retinoic acid.

The term "retinol" as used herein includes the following isomers of retinol: all-trans-retinol, 13-cis-retinol, 11-cis-retinol, 9-cis-retinol, 3,4-didehydro-retinol. Preferred isomers are all-trans-retinol, 13-cis-retinol, 3,4-didehydro-retinol, 9-cis-retinol, 9-cis-retinol. Most preferred is all-trans-retinol, due to its wide commercial activity.

Retinyl ester is an ester of retinol. The term "retinol" has been defined above. Retinyl esters suitable for use in the present invention are C₁-C₃₀ esters of retinol, preferably C₂-C₂₀ esters, and most preferably C₂, C₃, and C₁₆ esters because they are more commonly available. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecandate, retinyl laurate, retinyl tridecanoate, retinyl myristate, retinyl pentadecanoate, retinyl heptadecanoate, retinyl stearate, retinyl isosterate, retinyl nonadecanoate, retinyl arachidonate, retinyl behenate, retinyl linoleate, retinyl oleate, retinyl lactate, retinyl glycolate, retinyl hydroxy caprylate, retinyl hydroxy laurate, retinyl tartrate.

If present at all, the retinoids in the present invention may be present in an amount of from 0.001 % to 10%, preferably from 0.01 % to 1%, and most preferably from 0.01 % to 0.05% by weight of the final skin care composition.

The invention will now be further demonstrated by way of example only.

### Examples

Examples 1-4 demonstrate the preparation of suitable base materials for use in topical cosmetic compositions.

| Ingredients | Weight (g) | | | |
|---|---|---|---|---|
| | 1 | 2 | 3 | 4 |
| Fatty Acid* | 1.8 | 2.1 | 2.5 | 2.1 |
| Tween 60 | 1.1 | 1.1 | 0.58 | 1.1 |
| Glycolic acid | - | - | - | 0.4 |
| KOH | - | - | - | 0.14 |
| Water | 7.1 | 6.8 | 6.9 | 6.3 |

| | | | | |
|---|---|---|---|---|
| *Pristerene 4911, a roughly 50:50 blend of stearic and palmitic acids. | | | | |

### Methods of making the compositions

1. Hand shaking
   Powder or tablet made from any of formulations 1 to 4 (excluding water) was put in a screw top glass vial (ca 25ml volume). Boiling water was then added inside the vial to bring the total weight in the vial up to the level required for the full formulation, and the cap was replaced to seal the vial. The vial was then shaken for about 100 seconds, and then cooled down in an ambient environment or in a refrigerator. The total cooling time was about 3-5 minutes.
2. Vortex mixer
   Powder or tablet made from any of formulations 1 to 4 (excluding water) was put in a glass vial (ca 25ml volume). Boiling water was then added inside the vial, and the cap was replaced to seal the vial. This vial was then mixed by a vortex mixer for about 100 seconds. The vibration speed of the vortex mixer ranged ranged from 500-3000 rpm. The vial was then cooled in ambient environment or in a refrigerator. The total cooling time was about 3-5 minutes.
3. Ultrasonic heating and mixing
   Powder or tablet was from any of formulations 1-4 may be put in a plastic container, and water added to the container at ambient temperature. An ultrasonic heating and mixing device may then be used to heat the contents of the container to 80°C in 60 seconds. It may then be cooled in an ambient environment or in a refrigerator. The total cooling time was about 3-5 minutes.
4. Microwave heating and hand shaking
   Powder or tablet made from any of formulations 1 to 4 (excluding water) may be put in a glass vial (ca 25ml volume). Tap water can then be added to the vial, and the cap replaced to seal the vial. The vial may then be placed in a microwave and heated 15-25 seconds at 800 W power. The vial may then be shaken by hand for about 100 seconds, and then cooled in ambient environment or in a refrigerator. The total cooling time was about 3-5 minutes.

## Claims

1. A method of providing a topical base composition for use in the preparation of a cosmetic composition, especially a topical skin care composition, comprising:
(a) providing a cosmetic container;
(b) providing in the container an effective amount of a fatty acid material having a melting point in the range of 40°C to 80°C; and a nonionic surfactant; wherein the mixture of fatty acid material and nonionic surfactant is substantially anhydrous and is obtained by mere mixing of fatty acid material and nonionic surfactant;
(c) providing sufficient heated water to the container such that substantially all of the fatty acid material is solubilised to provide a fatty acid/nonionic surfactant base mixture; and
(d) agitating the contents of the container;
whereby a cream or lotion base is formed.

2. A method according to Claim 1, wherein the method is carried out in a cosmetic container by a consumer who seals the cosmetic container just prior to agitation.

3. A method according to Claim 1 or Claim 2, wherein the fatty acid material comprises a fatty acid blend.

4. A method according to Claim 3, wherein the fatty acid blend comprises a mixture of fatty acid moieties having chain lengths of C₈ to C₂₂.

5. A method according to Claim 4, wherein the fatty acid blend comprises any of pelargonic, lauric, myristic, palmitic, stearic, isostearic, oleic, linoleic, ricinoleic, arachidic, behenic or erucic acids.

6. A method according to Claim 5, wherein the fatty acid blend comprises at least 50% of stearic and/or palmitic acids.

7. A method according to any of the preceding claims wherein the resulting ratio of nonionic surfactant to fatty acid material after the provision of the heated water is in the ratio 1:1 to 1:20, more preferably 1:2 to 1:10.

8. A method according to any preceding claim wherein the heated water has a temperature of at least 85°C, more preferably at least 95°C.

9. A method according to any preceding claim wherein the container additionally contains other components necessary to provide a topical skin care cream or lotion.

10. A method according to Claim 9 wherein the resultant topical skin care cream or lotion has a pH in the range 3.5 to 6.

11. A method according to any preceding claim wherein ingredients of the fatty acid/nonionic surfactant base mixture are provided in the container in the form of a tablet, dried powder or amorphous mass.

12. A re-sealable cosmetic container containing an anhydrous composition comprising a mixture of a fatty acid material having a melting point in the range 40°C to 80°C and a nonionic surfactant.

13. A re-sealable cosmetic container according to Claim 12 additionally containing other components necessary to provide a skin care cream or lotion.

14. An anhydrous composition in the form of a tablet, powder or amorphous mass for dosing into a container for use in a method according to any of Claims 1-11.
